# EUROPEAN PATENT SPECIFICATION

(11) **EP 1 236 402 B1**
(45) Date of publication and mention of the grant of the patent: **20.01.2010**
(21) Application number: 02251391.5
(22) Date of filing: 27.02.2002
(51) Int. Cl.: A61Q 19/00, A61K 8/97

(54) **Compositions containing soy products**
Sojaprodukte enthaltende Zusammensetzungen
Compositions contenant des produits à base de soja

(30) Priority: 28.02.2001 US 796054; 28.02.2001 US 796293
(43) Date of publication of application: 04.09.2002
(60) Divisional application: 09075429.2
(73) Proprietor: JOHNSON & JOHNSON CONSUMER COMPANIES, INC., Skillman, New Jersey 08558 (US)
(72) Inventor: Liu, Jue-Chen, Belle Mead, New Jersey 08502 (US); Seiberg, Miri, New Jersey, NJ 08540 (US); Saliou, Claude, Gladstone, NJ 07934 (US); Miller, Jonathan D., Lawrenceville, NJ 08648 (US); Wu, Jeffrey M., Warrington, PA 18976 (US)
(74) Representative: Fisher, Adrian John

(56) References cited:
- HAFEZ Y S ET AL: "EFFECTS OF GAMMA IRRADIATION ON PROTEINS AND FATTY ACIDS OF SOYBEAN" JOURNAL OF FOOD SCIENCE, INSTITUTE OF FOOD TECHNOLOGISTS. CHICAGO, US, vol. 50, no. 5, 1985, pages 1271-1274, XP009005061 ISSN: 0022-1147
- JINGTIAN Y ET AL: "STUDIES OF SOY SAUCE STERILIZATION AND ITS SPECIAL FLAVOUR IMPROVEMENT BY GAMMA-RAY IRRADIATION" RADIATION PHYSICS AND CHEMISTRY, PERGAMON PRESS, OXFORD,, GB, vol. 31, no. 1-3, 1988, pages 209-213, XP009005444 ISSN: 0146-5724

## Description

### FIELD OF THE INVENTION

The present invention relates to legume products and the manufacture thereof.

### BACKGROUND OF THE INVENTION

Legume fruits contain high levels of proteins, lipids and carbohydrates. Consequently, legume fruits, such as soybeans, and compositions containing legume fruits are considered a great nutrient for human use. Legume fruits also contain compounds that inhibit protease activity. For example, two protein protease inhibitors were isolated from soybeans in the early 1940's, the Kunitz-type trypsin inhibitor (soybean trypsin inhibitor, STI) and the Bowman-Birk protease inhibitor (BBI). See, e.g., Birk, Int. J. Pept. Protein Res. 25:113-131 (1985) and Kennedy, Am. J. Clin. Neutr. 68:1406S-1412S (1998).

STI inhibits the proteolytic activity of trypsin by the formation of a stable stoichiometric complex. See, e.g., Liu, K., Chemistry and Nutritional value of soybean components. In: Soybeans, chemistry, technology and utilization. pp. 32-35 (Aspen publishers, Inc., Gaithersburg, MD, 1999). STI consists of 181 amino acid residues with two disulfide bridges and is roughly spherically shaped. See, e.g., Song et al., J. Mol. Biol. 275:347-63 (1998).

BBI is an 8 k-Da protein that inhibits the proteases trypsin and chymotrypsin at separate reactive sites. See, e.g., Billings et al., Pro. Natl. Acad. Sci. 89:3120-3124 (1992). STI and BBI are found only in the soybean seed, and not in any other part of the plant. See, e.g., Birk, Int. J. Pept. Protein Res. 25:113-131 (1985).

However, due to its natural origin, high levels of microorganisms are carried on the outside of legume fruits, such as soybeans. Consequently, decontamination processes such as heat, organic/aqueous solvent extraction, and high shear purification may be used to reduce such microorganism concentrations to allow it to be safe for human use, e.g., skin care applications. Applicants, however, have found that these processes, which frequently denature the active compounds in the soy, result in a compromised biological efficacy (e.g., a reduction in protease inhibitory activity) which is important for cosmetic and therapeutic uses to the skin, hair, and nails. Furthermore, such processes also can lead to instability of the soy product as well as to an undesirable odor and color generation. Therefore, there is a commercial need to develop a means to reduce the levels of microbials in soy products without compromising the biological benefits of such products.

The object of the present invention is to provide for a soy product (e.g., that can be used as an ingredient in a skin, hair, or nail care composition) that has reduced microbial content but maintains its protease inhibitory activity.

The present invention relates to legume products containing reduced microbial content that retains legume's beneficial biological activities and processes for obtaining such legume products.

### SUMMARY OF THE INVENTION

The present invention features legume products having trypsin inhibitory activity and reduced microbial content and methods of decontaminating such soy products. In one preferred embodiment, the legume product is a soy product.

Other features and advantages of the present invention will be apparent from the detailed description of the invention and from the claims

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. As used herein, all percentages are by weight unless otherwise specified.

As used herein, "trypsin inhibitory activity" means the ability of the legume product at a concentration of 0.1% (w/w) to inhibit the activity of the protease trypsin, as measured by the assay set forth below in Example 2. In one embodiment, the legume products of the present invention have a trypsin inhibitory activity of at least about 15%. In a further embodiment, the legume products of the present invention have a trypsin inhibitory activity of at least about 25%, such as at least about 50%.

As used herein, "thiol retention activity" means the ability of the legume product at a concentration of 1%(w/v) to inhibit smoke-induced loss of thiols, as measured by the assay set forth below in Example 3. In one embodiment, the legume products of the present invention have a thiol retention activity of at least about 75%. In a further embodiment, the legume products of the present invention have an thiol retention activity of at least about 90%, such as at least about 95%.

As used herein, "microbial content" means the amount of bacteria, fungi, and yeast present in the legume product. Examples of means to measure microbial content include, but are not limited to, the AOAC 986.23 Method as set forth in "Official Methods of Analysis of AOAC International," edited by Patricia Cunniff, Sixteenth Edition, 5^{th} Revision, 1999 (AOAC International) or the USP Method as set forth in "Official Compendia of Standards, USP 24 USP/NF 19", United States Pharmacopeial Convention, Inc., 2000 (Board of Trustees, United States Pharmacopeial Convention, Inc.).

"Objectionable microbial content" means the amount of bacteria, fungi, and yeast present in the legume product that are harmful to humans, including but not limited to coliform, E. Coli, Salmonella, thermophilic spores, Bacillus, Enterococcus, Staphylococcus, fecal streptococcus, and those listed in "Disinfection, sterilization, and preservation" 4th edition, Seymour S. Block, pp. 887-888 (1991, Lea & Febiger, Malvern, PA).

### Legume Product

What is meant by a "legume product" is a substance derived from a legume fruit. A legume is a plant from the family Leguminosae, which has a dehiscent fruit such as a bean, pea, or lentil. Examples of legumes, include but are not limited to, beans such as soybeans, lentil beans, peas, and peanuts.

The legume product may contain the entire legume fruit (e.g., the legume fruit ground into a powder) or only a portion of the legume (e.g., an extract of the legume). The legume product may be in the form of a fluid (e.g., a mixture of the legume fruit and water) or a solid (e.g., legume fruits powders). When in the form of a fluid, the term "legume product" refers to the solid constituents of the fluid derived from the legume.

The compositions of the present invention comprise a safe and effective amount of the legume product (e.g., soy product). In one embodiment, the composition contains from about 0.001% to about 50%, from about 1% to about 30%, of the legume product (e.g., soy product).

### Soy Product

What is meant by a "Soy Product" is a substance derived from the soybean. The soy product may contain only a portion of the soybean (e.g., an extract of the soybean such as a lipid reduced soybean powder or filtered soymilk) or may contain the entire soybean (e.g., a ground powder of the legume). The soy product may be in the form of a fluid (e.g., soymilk) or a solid (e.g., a soybean powder or soymilk powder). When in the form of a fluid, the term "soy product" refers to the solid constituents of the fluid that are derived from the soybean.

In one embodiment, the soy product is soybean powder. Soybean powder may be made by grinding dry soybeans. In one embodiment, the soybean powder has a average particle size of less than about 10 micrometers such as less than about 1 micrometer. In one embodiment, the soybean powder has a moisture content of less than about 10% such as less than about 5%. In one embodiment, the soybean powder is lyophilized.

In one embodiment, the soy product is soymilk or soymilk powder. Soymilk is a combination of solids derived from soybeans and water, the mixture of which has some or all of the insoluble constituents filtered off. Soymilk powder is evaporated soymilk, which in one embodiment, is in a lyophilized or spray-dried form. Procedures for manufacturing soymilk include, but are not limited to, the following three procedures. First, soymilk may be made by placing soybeans into water to allow them to absorb the water. The swelled beans are then ground and additional water is then added. The mixture may then filtered to remove any insoluble residue. Second, soymilk may also be prepared from soybean powder. Soybean powder is thoroughly mixed with water (e.g., for at least one hour), which may then be followed by a filtration process to remove insoluble residues. Third, soymilk can also be reconstituted from soymilk powder by adding water. In one embodiment, soymilk comprises from between about 1% to about 50%, by weight (e.g., from about 5% to about 20%, by weight) of solids from the soybean.

### Anti-microbial Treatment of Legume Product

As discussed above, the surface of legume fruits often contain high levels of microorganisms. Thus, prior to use by humans, the legume product needs to be treated to reduce or eliminate such microorganisms.

In one embodiment, the legume products of the present invention have a total microbial content of less than about 10,000 colony-forming units ("cfu") per gram. In a further embodiment, the soy products of the present invention have a microbial content of less than about 1,000 cfu per gram (such as less than about 100 cfu per gram) of the legume product.

In one embodiment, the legume products of the present invention have a total objectionable microbial content of less than 300 cfu per gram such as less than 150 cfu per gram. In a further embodiment, the legume products of the present invention have an undetectable amount of any objectionable microbials for at least one gram (e.g., at least ten grams) of legume product.

In one embodiment, the legume product is exposed to gamma irradiation. In a further embodiment, the legume product is exposed to between about 2 to about 30 kGy of gamma irradiation, such as between about 5 and about 10 kGy of gamma irradiation. Applicants have unexpectedly found that such treatment reduces the microbial content of the legume product, while maintaining its biological activity (e.g., serine protease inhibitory activity). Applicants have also found that treatment of legume products with gamma irradiation maintains the cosmetic elegance of the legume product, such as maintained its natural colors and did not induce significant malodors.

Other anti-microbial processes that also maintain the protease inhibitory activity of the legume product that can be practiced alone or in combination with gamma irradiation, include, but are not limited to, exposure to x-rays, high energy electron or proton beams, ultraviolet radiation, hydrostatic pressure, and addition of chemical agents possessing antimicrobial activity, and combinations thereof. A complete list of methods for microbial content reduction is set forth in "Disinfection, sterilization, and preservation" 4th edition, Seymour S. Block, pp. 887-888 (1991, Lea & Febiger, Malvern, PA).

Applicants have found that processes using thermal treatment may result in a substantial loss in protease inhibitory activity and, thus, should be used with caution. For example, applicants have found that heating soymilk to 100°C for only 10 minutes reduced the trypsin inhibitory activity of the soymilk from 86% (when maintained at 4°C) to 46%. Applicants have found that heating soymilk can also result in a change of the color or odor of the soybean product.

### Mineral Water

The legume product (e.g., soymilk) and compositions of the present invention may be prepared using a mineral water. In one embodiment, the mineral water has a mineralization of at least about 200 mg/L (e.g., from about 300 mg/L to about 1000 mg/L). In one embodiment, the mineral water comprises at least about 10 mg/L of calcium and/or at least about 5 mg/L of magnesium.

### Example 1: Gamma Irradiation of Legume Product

Applicants have found that soymilk powder prior to any antimicrobial processing such as gamma irradiation has high levels microbial content, ranging from up to 50,000 cfu per gram. Such products were also found to have detectable levels of objectionable microbial content, such as fecal streptococci, at levels up to 20,000 cfu per gram.

Applicants have exposed various amounts (e.g., from about 1 g to about 200 kg) of soymilk powder to gamma irradiation varying from 1 kGy to 16 kGy. The dose or gamma irradiation needed for a reduction a total microbial content to less than about 100 cfu per gram was found to be about 10 kGy. The dose for one log reduction for fecal streptococci is determined to be about 3 kGy and a dose of about 5 kGy was found to consistently reduce this microbial content within a 10 gram sample of soymilk powder to undetectable levels. However, the amount of gamma irradiation used on the legume product will ultimately be determined by the microbial content and size of the soy product to be so treated.

### Example 2: Trypsin Inhibitory Activity of Legume Product

The inhibition of trypsin-induced cleavage of a fluorescent casein peptide was measured using the EnzChek™ protease assay kit, following manufacturer's instructions (EnzChek™ Protease Assay Kits Product Information, Revised 3/15/99; Molecular Probes, Eugene OR). In summary, various soy preparations were first diluted in 1X digestion buffer (provided in kit) and incubated at different concentrations with 1000 units of trypsin (Sigma, St. Louis, MO) dissolved in 1X digestion buffer. A pure serine protease inhibitor (soybean trypsin inhibitor, from Sigma, St. Louis, MO) was used as a positive control at 0.1, 0.01%, and 0.001% w/v. Then, 1.0 mg/ml stock solution of BODIPY FL casein was prepared by adding 0.2 mL of deionized water to the vials supplied with this substrate (provided in kit), then made to a final working concentration of 10 microgram/ml in digestion buffer. Following incubation of the trypsin, with or without the test material, with the BODTPY fluorescent casein substrate at room temperature for one hour, fluorescence was measured (excitation 485 nm /emission 530 nm) on a SpectraMax^{®} Gemini microtiter plate reader (Molecular Devices Corporation, Sunnyvale, CA) using Softmax^{®} Pro 3.0 software (Molecular Devices Corporation). Each experiment was performed in three replicates and was repeated twice.

This assay was performed on soy products processed seven different ways. Example A was soybeans ground into powder (Sunlight Foods Corporation, Taipei County, Taiwan, R.O.C.). Example B was soybean powder of Example A exposed to about 8-15 kGy of gamma irradiation. Example C was soybean powder in which the oil in the soybean powder was removed by extraction (Soyafluff® 200W from Central Soya Company, Inc., Fort Weyne, IN). Example D was soymilk powder made with dehulled soybeans and water that was subsequently filtered and heated and spray dried (Devansoy Farms, Carroll, Iowa) and exposed to between about 7 - 9 kGy of gamma irradiation. Example E was soymilk powder obtained by mixing soy beans and water, heating the mixture overnight, and adding 1,3-butylene glycol to the mixture (Flavosterone SB from Ichimaru Pharcos Co., Ltd, Gifu Japan). Example F was soymilk powder obtained by mixing soy beans and water, heating the mixture overnight, and subsequently adding ethanol to the mixture (Flavosterone SE from Ichimaru Pharcos Co., Ltd, Gifu Japan). Example G was an extract of soy proteins (Vegeseryl HGP LS 8572 from Laboratories Serobiologiques S.A., Pulnoy, France). These soy products were compared to Soy Trypsin Inhibitor (STI) (Sigma).

The percent inhibition of trypsin cleavage of the substrate by the different soy preparations was calculated using Microsoft Excel™ and is reported in Table 1.

**Table 1**

| Tested Product | Concentration | % Inhibition of Trypsin |
|---|---|---|
| STI | 0.01 | 43.0 |
| STI | 0.1 | 76.1 |
| Example A | 0.01 | 32.8 |
| Example A | 0.1 | 67.1 |
| Example B | 0.01 | 31.5 |
| Example B | 0.1 | 67.2 |
| Example C | 0.01 | 22.7 |
| Example C | 0.1 | 36.2 |
| Example D | 0.01 | 8.92 |
| Example D | 0.1 | 17.4 |
| Example E | 0.01 | 7.83 |
| Example E | 0.1 | 10.8 |
| Example F | 0.01 | 4.87 |
| Example F | 0.1 | 5.99 |
| Example G | 0.1 | 6.85 |

As shown in Table 1, STI can inhibit trypsin-induced cleavage in a dose response manner. Example A, which is soybean powder, also significantly inhibited trypsin activity. Further gamma irradiation of the soybean powder (i.e., Example B), while reducing the microbial content of the soybean powder, unexpectedly did not significantly impact the trypsin inhibition activity of the soybean powder. The heat and/or extraction processing of Examples C-G, however, did significantly reduce the trypsin inhibitory activity of the soybean powder.

### Example 3: Thiol Retention Activity of Legume Product

The ability of soy powder to prevent smoke-induced loss of thiols was evaluated in normal human dermal fibroblasts (Clonetics, San Diego, CA). Thiols, chiefly glutathione, are part of the endogenous cellular antioxidant defense system. Glutathione serves as a redox buffer, thereby, maintaining the balance between oxidants and antioxidants. Glutathione is also the preferred substrate for several enzymes such as the glutathione peroxidases (decomposing peroxides) and the glutathione-S-transferases (a major group of detoxification enzymes). See, A. Meister, Cancer Res. 54:1969s-1975s (1994).

Cutaneous antioxidants (both enzymatic and non-enzymatic), including glutathione, are depleted after UV or ozone exposure. See, M. J. Connor and L. A. Wheeler, Photochem. Photobiol. 46:239-246 (1987) and R. M. Tyrrell and M. Pidoux, Photochem. Photobiol. 47:405-412 (1988). In cell culture models, low intracellular glutathione (GSH) levels lead to a higher UV radiation sensitivity. Topical application of cysteine derivatives on rat skin has been shown to protect against UV radiation-induced photodamage; this benefit was correlated with an increase in GSH synthesis. See, L. T. van den Broeke and G. M. J. Beijersbergen van Henegouwen, J. Photochem. Photobiol. B Biol. 27:61-65 (1995); K. Hanada, et al., J. Invest. Dermatol. 108:727-730 (1997); and D. P. T. Steenvoorden, et al., Photochem Photobiol. 67:651-656 (1998). Consequently, glucothione is a major endogenous antioxidant, highly responsive against environmental challenges, able to regulate the tone and the wrinkling of skin, as well as treat external aggression.

In this experiment, normal human neonatal dermal fibroblasts seeded in 24-well format Transwell inserts (Corning Costar, Cambridge, MA) were incubated with media containing various concentrations of soymilk powder (gamma irradiated at about 5 kGy) for 24 hours prior to exposure with either placebo (mock) or cigarette smoke (1 cigarette, BASIC Full Flavor 100's cigarettes, Philip Morris, Richmond, VA) for 10 minutes.

Prior to smoke exposure, the medium above the inserts containing the soy product was removed, and the cells were washed 3 times with Dulbecco's Phosphate-Buffered Saline (Life Technologies, Gaithersburg, MD) before being smoke-exposed with only media below the inserts. Immediately after exposure, the cells were incubated for another 24-hour period with the previous medium. The cells were washed again, 5 times with Dulbecco's Phosphate-Buffered Saline, and intracellular thiols were then measured by adding 60 µM monobromobimane (Molecular Probes, Eugene, OR, USA) to the cells and incubating at 37°C for 30 minutes before the fluorescence reading. In the presence of thiols, the monobromobimane becomes fluorescent. This fluorescence was measured using a CytoFluor® Fluorescence Plate Reader (PerSeptive Biosystems, Framingham, MA, USA) set with the following filter combination: excitation at 360nm and emission at 460nm.

The results of this experiment are set-forth in Table 2.

**Table 2**

| | Soymilk Powder concentration (weight %) | Thiols (Percent of Thiols contained in No Smoke Group; Mean ± S.E.M.) |
|---|---|---|
| No Smoke | 0 | 100 ± 6.71 |
| Smoke (10 min.) | 0 | 65.38 ± 7.16 |
| | 0.5 | 91.24 ± 14.25 |
| | 1 | 95.39 ± 4.52 |
| | 2 | 106.92 ± 17.06 |

These results indicate that gamma irradiated soymilk powder surprisingly afforded a protection against smoke-induced loss of thiols (data represent the mean ± Standard of the mean of replicates from 3 independent experiments).

It is understood that while the invention has been described in conjunction with the detailed description thereof, that the foregoing description is intended to illustrate and not limit the scope of the invention, which is defined by the scope of the appended claims.

Other aspects, advantages, and modifications are within the claims.

## Claims

1. A soy product having a trypsin inhibitory activity of at least 50% and a microbial content less than 10,000 cfu per gram;
wherein said soy product has been exposed to gamma irradiation;
wherein said soy product is selected from soymilk powder or soybean powder having an average particle size of less than 10 micrometers; and
wherein said soy product has a thiol retention activity of at least 75%.

2. A soy product according to claim 1 having a trypsin inhibitory activity of at least 15% and a microbial content less than 1000 cfu per gram.

3. A soy product of claim 2, said soy product having a harmful to humans microbial count less than 1000 cfu per gram.

4. A soy product of claim 1 or claim 2, said soy product having a harmful to humans microbial count less than 150 cfu per gram.

5. A soy product of claim 4, said soy product having a microbial content of less than 100 cfu per gram.

6. A soy product of any preceding claim, said soy product having no detectable harmful to humans microbial content per gram.

7. A soy product of any preceding claim, wherein said soy product is soymilk powder.

8. A soy product of any of claims 1 to 7, wherein said soy product is soybean powder.

9. A method of producing a decontaminated soy product, wherein said soy product is selected from soymilk powder or soybean powder having an average particle size of less than 10 micrometers, said soy product has a trypsin inhibitory activity of at least 50%, a microbial content less than 10,000 cfu per gram and a thiol retention activity of at least 75%; said method comprising exposing said soy product to gamma irradiation.

10. The method of claim 9, wherein said soy product is exposed to 5 to 16 kGy gamma irradiation.

11. The method of claim 9 or claim 10, wherein said soy product is soymilk powder.

12. The method of claim 9 or claim 10, wherein said soy product is soybean powder.

## Patentansprüche

1. Sojaprodukt mit einer trypsinhemmenden Aktivität von wenigstens 50% und einem Mikrobengehalt von weniger als 10.000 cfu pro Gramm;
wobei das Sojaprodukt Gammastrahlung ausgesetzt gewesen ist; wobei das Sojaprodukt ausgewählt ist aus Sojamilchpulver oder Sojabohnenpulver mit einer durchschnittlichen Teilchengröße von weniger als 10 Mikrometern; und
wobei das Sojaprodukt eine Thiolretentionsaktivität von wenigstens 75% besitzt.

2. Sojaprodukt nach Anspruch 1 mit einer trypsinhemmenden Aktivität von wenigstens 15% und einem Mikrobengehalt von weniger als 1.000 cfu pro Gramm.

3. Sojaprodukt nach Anspruch 2, wobei das Sojaprodukt eine Zahl von für Menschen schädlichen Mikroben von weniger als 1.000 cfu pro Gramm besitzt.

4. Sojaprodukt nach Anspruch 1 oder Anspruch 2, wobei das Sojaprodukt eine Zahl von für Menschen schädlichen Mikroben von weniger als 150 cfu pro Gramm besitzt.

5. Sojaprodukt nach Anspruch 4, wobei das Sojaprodukt einen Mikrobengehalt von weniger als 100 cfu pro Gramm besitzt.

6. Sojaprodukt nach einem vorangehenden Anspruch, wobei das Sojaprodukt keinen nachweisbaren Gehalt an für Menschen schädlichen Mikroben pro Gramm besitzt.

7. Sojaprodukt nach einem vorangehenden Anspruch, wobei das Sojaprodukt Sojamilchpulver ist.

8. Sojaprodukt nach einem der Ansprüche 1 bis 7, wobei das Sojaprodukt Sojabohnenpulver ist.

9. Verfahren zur Herstellung eines dekontaminierten Sojaproduktes, wobei das Sojaprodukt ausgewählt ist aus Sojamilchpulver oder Sojabohnenpulver mit einer durchschnittlichen Teilchengröße von weniger als 10 Mikrometern, das Sojaprodukt eine trypsinhemmende Aktivität von wenigstens 50%, einen Mikrobengehalt von weniger als 10.000 cfu pro Gramm und eine Thiolretentionsaktivität von wenigstens 75% besitzt; wobei das Verfahren das Bestrahlen des Sojaproduktes mit Gammastrahlung umfasst.

10. Verfahren nach Anspruch 9, wobei das Sojaprodukt 5 bis 16 kGy Gammastrahlung ausgesetzt wird.

11. Verfahren nach Anspruch 9 oder Anspruch 10, wobei das Sojaprodukt Sojamilchpulver ist.

12. Verfahren nach Anspruch 9 oder Anspruch 10, wobei das Sojaprodukt Sojabohnenpulver ist.

## Revendications

1. Produit à base de soja présentant une activité inhibitrice de la trypsine d'au moins 50 % et comprenant une teneur en microbes inférieure à 10 000 unités formant des colonies par gramme ;
➢ dans lequel ledit produit à base de soja a été exposé à une irradiation gamma ;
➢ dans lequel ledit produit à base de soja est sélectionné parmi du lait de soja en poudre ou de la poudre de soja présentant une taille de particule moyenne inférieure à 10 micromètres ; et
➢ dans lequel ledit produit à base de soja présente une activité de rétention de thiol d'au moins 75 %.

2. Produit à base de soja selon la revendication 1 présentant une activité inhibitrice de la trypsine d'au moins 15 % et comprenant une teneur en microbes inférieure à 1000 unités formant des colonies par gramme.

3. Produit à base de soja selon la revendication 2, ledit produit à base de soja comprenant une teneur en microbes nuisibles pour l'homme inférieure à 1000 unités formant des colonies par gramme.

4. Produit à base de soja selon la revendication 1 ou la revendication 2, ledit produit à base de soja comprenant une teneur en microbes nuisibles pour l'homme inférieure à 150 unités formant des colonies par gramme.

5. Produit à base de soja selon la revendication 4, ledit produit à base de soja comprenant une teneur en microbes inférieure à 100 unités formant des colonies par gramme.

6. Produit à base de soja selon l'une quelconque des revendications précédentes, ledit produit à base de soja ne comprenant pas de teneur détectable en microbes nuisibles pour l'homme par gramme.

7. Produit à base de soja selon l'une quelconque des revendications précédentes, dans lequel ledit produit à base de soja est du lait de soja en poudre.

8. Produit à base de soja selon l'une quelconque des revendications 1 à 7, dans lequel ledit produit à base de soja est de la poudre de soja.

9. Méthode de production d'un produit à base de soja décontaminé, dans laquelle ledit produit à base de soja est sélectionné parmi du lait de soja en poudre ou de la poudre de soja présentant une taille de particule moyenne inférieure à 10 micromètres, ledit produit à base de soja présentant une activité inhibitrice de la trypsine d'au moins 50 %, une teneur en microbes inférieure à 10 000 unités formant des colonies par gramme et une activité de rétention de thiol d'au moins 75 % ; ladite méthode comprenant l'exposition dudit produit à base de soja à une irradiation gamma.

10. Méthode selon la revendication 9, dans laquelle ledit produit à base de soja est exposé à une irradiation gamma de 5 à 16 kGy.

11. Méthode selon la revendication 9 ou la revendication 10, dans laquelle ledit produit à base de soja est du lait de soja en poudre.

12. Méthode selon la revendication 9 ou la revendication 10, dans laquelle ledit produit à base de soja est de la poudre de soja.
